(19) Europäisches Patentamt
European Patent Office
Office européen des brevets

(11) Publication number : **0 638 614 A1**

# (12) EUROPEAN PATENT APPLICATION

(21) Application number : **94305325.6**

(22) Date of filing : **20.07.94**

(51) Int. Cl.[6] : **C09B 47/08,** C07D 487/22, // (C07D487/22, 259:00, 209:00, 209:00, 209:00, 209:00)

(30) Priority : **12.08.93 GB 9316820**

(43) Date of publication of application : **15.02.95 Bulletin 95/07**

(84) Designated Contracting States : **CH DE FR GB IT LI**

(71) Applicant : **ZENECA LIMITED**
**15 Stanhope Gate**
**London W1Y 6LN (GB)**

(72) Inventor : **Reynolds, Stephen James**
**Flat 9,**
**Princess Court,**
**38 Circular Road**
**Withington, Manchester M20 3LP (GB)**
Inventor : **White, Raymond Lesley**
**22 Northlands**
**Radcliffe, Manchester M26 4GB (GB)**

(74) Representative : **Giles, David Eric et al**
**Intellectual Property Group**
**Zeneca Specialties**
**P.O. Box 42**
**Hexagon House**
**Blackley**
**Manchester M9 8ZS (GB)**

(54) **Phthalocyanines.**

(57) A phthalocyanine of Formula (1) :

$$(R)_a M_k Pc\ (O\text{-}R^1)_b (O\text{-}R^2)_c X_d \qquad \text{Formula (1)}$$

wherein :

$M_kPC$ is a phthalocyanine nucleus of the Formula (2) :

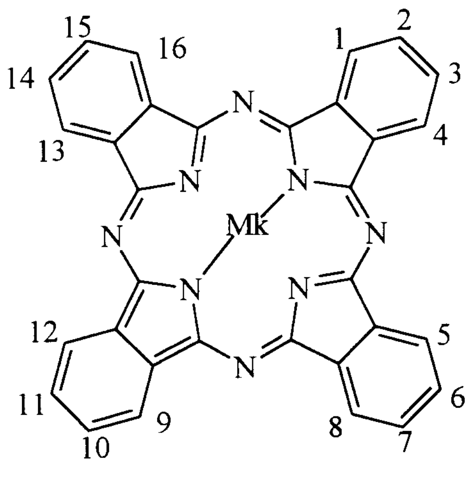

Formula(2)

in which

M is a metal atom ;

k is inverse of $\frac{1}{2}$ valency of M ;

R is a heterocyclic compound or a tertiary amine ;

each $R^1$ independently is an aryl or heterocyclic radical ;

each $R^2$ independently is an alkyl or cycloalkyl ;

each X independently is -H or halogen ;

a is 1 or 2 ;

b is an integer from 1 to 8 ;

EP 0 638 614 A1

c is 0 or is an integer from 1 to 4 ;
d is an integer from 4 to 15 ; and
$b+c+d = 16$.

The phthalocyanines are useful for absorbing electomagnetic radiation and may be used for optical data storage, as liquid crystals, as singlet oxygen generators, in ink jet printing, in security printing, as charged generating materials and as non-linear absorbers.

This invention relates to phthalocyanines, particularly to aryloxy substituted phthalocyanines which exhibit strong, narrow band absorption at 600 to 800nm.

According to the present invention there is provided a phthalocyanine of Formula (1):

$$(R)_aM_kPc(O\text{-}R^1)_b(O\text{-}R^2)_cX_d \qquad \text{Formula (1)}$$

wherein:

$M_kPc$ is a phthalocyanine nucleus of the Formula (2):

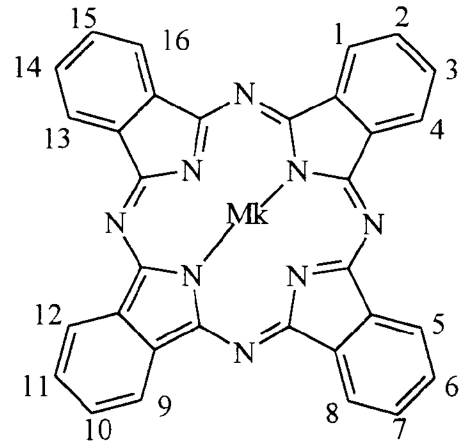

Formula(2)

in which

| | |
|---|---|
| M | is a metal atom; |
| k | s inverse of ½ valency of M; |
| R | s a heterocyclic compound or a tertiary amine; |
| each $R^1$ | ndependently is an aryl or heterocyclic radical; |
| each $R^2$ | ndependently is an alkyl or cycloalkyl radical; |
| each X | ndependently is -H or halogen; |
| a | s 1 or 2; |
| b | s an integer from 1 to 8; |
| c | s 0 or is an integer from 1 to 4; |
| d | s an integer from 4 to 15; and |

b+c+d = 16.

M is preferably a transition metal, more preferably a metal from the first transition row of the Periodic Table and especially iron, or cobalt. Where M is iron, or cobalt it is preferably present in the divalent state i.e. as $Fe^{II}$, or $Co^{II}$. It is especially preferred that M is $Fe^{II}$.

The heterocyclic compound represented by R preferably has one or more heteroatom(s) each of which has at least one pair of electrons and is capable of complexing with the central metal, M. The heterocyclic compound represented by R preferably contains at least one nitrogen, sulphur or oxygen atom as the heteroatom or contains a combination of such atoms. Suitable heterocyclic compounds include pyridine, quinoline, isoquinoline, bipyridyl, pyrazine, pyrimidine, pyridazine, triazine, thiophene, furan, pyrrole, pyrazole, imidazole, thiazole, isothiazole, thiadiazole, oxazole, isooxazole, pyran, oxazine, morpholine, indole, bipyridyl each of which may be optionally substituted. Suitable optional substituents for the heterocyclic groups represented by R may be selected from $C_{1-8}$-alkyl, $C_{1-8}$-alkoxy, $-NH_2$, $-NHC_{1-6}$-alkyl, $-N(C_{1-6}\text{-alkyl})_2$, -Cl, -F, -Br, -CN, $-CF_3$, $-NO_2$, -OH, $C_{1-4}$-alkylphenyl, $C_{1-8}$-alkyl$C_{1-8}$-alkoxy, $C_{1-8}$-alkoxy$C_{1-8}$-alkyl and $C_{1-8}$-alkoxy$C_{1-8}$-alkoxy. The heterocyclic compound represented by R is preferably an optionally substituted pyridine, quinoline, isoquinoline, bipyridyl and pyrazine, more preferably an optionally substituted pyridine, quinoline, isoquinoline, bipyridyl and pyrazine which is unsubstituted or which carry $C_{1-8}$-alkyl, $C_{1-4}$-alkylphenyl, -CN or $-N(C_{1-6}\text{-alkyl})_2$ substituents and especially pyridine, isoquinoline, bipyridyl and pyridines substituted in the 3- and/or 4- and/or 5 positions by t-butyl, benzyl, -CN, $-N(CH_3)_2$ or $-N(C_2H_5)_2$.

The tertiary amine represented by R is preferably $N(C_{1-8}\text{-alkyl})_3$ or aryl$N(C_{1-8}\text{-alkyl})_2$, more preferably phenyl$N(C_{1-4}\text{-alkyl})_2$ and especially phenyl$N(CH_3)_2$, phenyl$N(C_2H_5)_2$ or phenyl$N(C_4H_9)_2$.

R is preferably a heterocyclic compound.

Where the heterocyclic group represented by R has more than one heteroatom capable of complexing with the central metal, M, polymeric compounds of the following general Formula (3) may be formed:

$$\left[\begin{array}{l} R^3 \\ M_kPc(O-R^1)_b(O-R^2)_cX_d \\ R^3 \\ M_kPc(O-R^1)_b(O-R^2)_cX_d \end{array}\right]_{\varepsilon}$$

Formula (3)

wherein:

$R^3$ is a heterocyclic compound having two or more heteroatoms in which at least two heteroatoms are capable of complexing with the central metal;

$\varepsilon$ is equal to or greater than 1 and

$M_kPc$, $R^1$, $R^2$, X, b, c and d are as hereinbefore defined, such polymeric compounds form a further feature of the present invention.

The aryl and heterocyclic radicals represented by $R^1$ are each independently selected from optionally substituted mono- or bi-cyclic aromatic radicals or from optionally substituted mono- or bi-cyclic heterocyclic radicals. Examples of suitable aryl and heterocyclic radicals are phenyl, phenylene, especially 1,2-phenylene, naphthyl especially 2-naphthyl, pyridyl, quinolinyl, isoquinolinyl, thiophenyl, furanyl, pyrimidyl, thiazolyl, isothiazolyl, benzothiazolyl and benzoisothiazolyl each of which may be optionally substituted. Where the aryl radical represented by $R^1$ is 1,2-phenylene this is attached to the phthalocyanine nucleus via two oxygen atoms. The two oxygen atoms occupy the 1,2-positions of each phenylene radical and are preferably attached to the phthalocyanine nucleus via the 2- and 3- and/or 6- and 7- and/or the 10- and 11- and/or the 14- and 15-positions. $R^1$ is preferably optionally substituted phenyl, 2-naphthyl and pyridyl, especially optionally substituted phenyl and 2-naphthyl.

Suitable substituents for the aryl or heterocyclic radicals represented by $R^1$ may be selected from $C_{4-12}$-alkyl, preferably $C_{4-8}$-alkyl and especially branched chain alkyl such as t-butyl and t-octyl; $C_{5-20}$-alkoxy; $C_{4-8}$-cycloalkyl; aryl such as phenyl; -CN; $-N(C_{1-10}\text{-alkyl})_2$ and $C_{1-4}$-alkylaryl, preferably benzyl and especially those substituents which aid the solubility of the phthalocyanine in organic solvents.

The alkyl radical represented by $R^2$ is preferably optionally substituted $C_{1-20}$-alkyl, more preferably $C_{1-12}$-alkyl and especially $C_{1-8}$-alkyl. The cycloalkyl radical represented by $R^2$ is preferably optionally substituted $C_{3-10}$-cycloalkyl, more preferably $C_{4-8}$-cycloalkyl and especially cyclohexyl.

Suitable optional substituents for the alkyl and cycloalkyl groups represented by $R^2$ are any of those listed above as suitable substituents for the heterocyclic groups represented by R.

Each halogen represented by X is independently selected from fluoro, chloro, bromo and iodo, preferably chloro and bromo and more preferably chloro.

a is preferably 2.

b is preferably an integer from 4 to 8 and more preferably 4 or 8.

c is preferably 0 or 4, more preferably 0.

d is preferably an integer from 4 to 12, more preferably from 8 to 12.

In phthalocyanines of Formula (1) it is preferred that the O- $R^1$ groups are substituted in the 2- and 3-, the 6- and 7-, the 10- and 11- and the 14- and 15-positions of the phthalocyanine nucleus of Formula (2). Thus where b is 8 the O-$R^1$ groups preferably occupy the 2-, 3-, 6-, 7-, 10-, 11-, 14- and 15-positions. Where b is 4 one position of each of the four pairs of positions i.e. 2- or 3- and 6- or 7- and 10- or 11- and 14- or 15- are preferably occupied.

A first preferred sub-group of phthalocyanines of Formula (1) is that in which

| | |
|---|---|
| R | is pyridine or pyridine substituted in the 3- and/or 4- and/or 5-positions by $C_{1-4}$-alkyl, $C_{1-4}$-alkylphenyl, -CN, or $-N(C_{1-4}\text{-alkyl})_2$, or isoquinoline; |
| M | is $Fe^{II}$, or $Co^{II}$; |
| each $R^1$ | independently is phenyl or naphth-2-yl optionally substituted by one or more straight or branched chain $C_{4-8}$-alkyl group; |
| each X | independently is -H or halogen; |
| a | is 2; |

b is 8;
c is 0; and
d is 8.

A second preferred sub group of phthalocyanines of Formula (1) is that in which

R is pyridine or pyridine substituted in the 3- and/or 4- and/or 5-positions by $C_{1-4}$-alkyl, $C_{1-4}$-alkylphenyl, -CN or -N($C_{1-4}$-alkyl)$_2$; or isoquinoline;
M is $Fe^{II}$ or $Co^{II}$;
each $R^1$ independently is phenyl or naphth-2-yl optionally substituted by one or more straight or branched chain $C_{4-8}$-alkyl group;
each $R^2$ independently is $C_{1-8}$-alkyl or $C_{4-8}$-cycloakyl;
each X independently is -H or halogen;
a is 2;
b is 4;
c is 4;
d is 8.

A third preferred sub-group of phthalocyanines of Formula (1) is that in which

R is pyridine or pyridine substituted in the 3- and/or 4- and/or 5-positions by $C_{1-4}$ -alkyl, $C_{1-4}$-alkylphenyl, -CN or -N($C_{1-4}$-alkyl)$_2$, or isoquinoline;
M is $Fe^{II}$ or $Co^{II}$;
each $R^1$ independently is phenyl or naphth-2-yl optionally substituted by one or more straight or branched chain $C_{1-4}$ -alkyl group;
each X independently is -H or halogen;
a is 2;
b is 4;
c is 0;
d is 12

Particularly preferred phthalocyanines of Formula (1) are those of the first, second and third sub-groups in which M is $Fe^{II}$.

Where X = Cl, d is preferably 4.

Especially preferred phthalocyaninies of Formula (1) are octa-4,5(4-t-octylphenoxy) ironphthalocyanine (R)$_2$, tetra-4-(2,4-di-t-butylphenoxy) ironphthalocyanine (R)$_2$ and tetra-4-(2,6-di-t-butylphenoxy)-tetra-5-(4-t-butylphenoxy)ironphthalocyanine (R)$_2$ in which R is pyridine or substituted pyridine.

The phthalocyanines of Formula (1) of particular interest are those which have a maximum absorbance peak ($\lambda$max) in the 640 to 750nm region of the electromagnetic spectrum, preferably in the region 650 to 700nm and especially in the region 650 to 680 and which have solubility of at least 1% in organic liquids. The organic liquids are selected from aliphatic, alicyclic and aromatic hydrocarbons, ketones, ethers, halogenated aliphatic and aromatic hydrocarbons, amides and substituted amides. Particular examples of suitable organic liquids are toluene, cyclohexanone, methylethylketone, tetrahydrofuran (THF), chloroform, dichloromethane and dimethylformamide.

The phthalocyanines of Formula (1) may be prepared by firstly reacting a 1,2-dicyanobenzene of Formula (3):

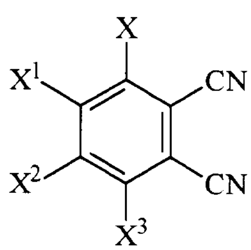

Formula (3)

wherein:
X, $X^1$, $X^2$ and $X^3$ each independently is H or halogen, provided at least one of X, $X^1$, $X^2$ or $X^3$ is halogen, with

an optionally substituted phenol, $R^1OH$ or an optionally substituted alkanol or an optionally substituted cycloalkanol, $R^2OH$ to form a compound of Formula (4):

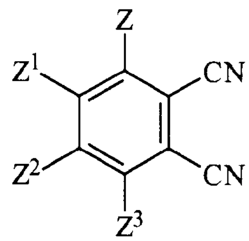

Formula(4)

wherein
Z, $Z^1$, $Z^2$ and $Z^3$ each independently is H, halogen, $-OR^1$ or $-OR^2$, provided at least one of Z, $Z^1$, $Z^2$ or $Z^3$ is $-OR^1$ or $OR^2$ in which $R^1$ and $R^2$ are as hereinbefore defined.

The compound of Formula (4), or a mixture of different compounds of Formula (4), is then reacted with an appropriate metal or metal salt, optionally in an inert liquid at elevated temperature to form a phthalocyanine of Formula (5).

$$M_kPc\ (O\text{-}R^1)_b\ (O\text{-}R^2)_cX_d \qquad \text{Formula (5)}$$

wherein $M_kPc$, $R^1$, $R^2$, X, b, c and d are as hereinbefore defined.

Where $R^1$ is 1,2-phenylene the compound of Formula (3) may be reacted with 1,2-dihydroxybenzene wherein two adjacent groups represented by X to $X^3$ are replaced.

In the above reaction appropriate metal salts include metal halides, metal oxides and metal hydroxides. For example where H is Fe, $FeCl_3.6H_20$ may be used as metal salt, where M is Cu, $CuCl_22.H_20$ may be used as metal salt and where M is Co, $CoCl_26.H_20$ may be used as metal salt.

Suitable inert liquids include halohydocarbons such as 1-chloronaphthalene, 1,2,4-trichlorobenzene, nitrohydrocarbons such as nitrobenzene, and diols such as ethylene glycol.

Suitable elevated temperatures are from 80 to 300°C, preferably from 100 to 250°C and especially from 150 to 210°C.

The compounds of Formula (1) may be prepared by reaction of a compound of Formula (5) with a heterocyclic compound or a tertiary amine represented by R. The process may optionally be carried out in an inert liquid medium and at an elevated temperature. This reaction is preferably carried out in the heterocyclic compound or the tertiary amine, however, when an inert liquid medium is used this is preferably an organic liquid and more preferably is selected from aromatic hydrocarbons such as toluene, aliphatic halocarbons such as dichloromethane, aromatic halocarbons such as 1-chloronaphthalene and 1,2,4-trichlorobenzene, ethers such as tetrahydrofuran. Suitable elevated temperatures are from 50 to 280°C preferably from 100 to 240°C and especially from 100 to 200°C.

The phthalocyanine compounds of the present invention are useful for absorbing electromagnetic radiation and may be used for optical data storage, as liquid crystals, as singlet oxygen generators, in ink jet printing particulary hot melt ink jet printing, in security printing and as charge generating materials and as non-linear absorbers.

The invention is further illustrated by the following examples in which all parts and percentages are by weight unless otherwise indicated.

Example 1

Preparation of octa-4,5-(4-t-octylphenoxy)iron(II) phthalocyanine. dipyridine

i) Preparation of 4,5-Dichlorophthalicanhydride

4,5-Dichlorphthalic acid (250.76g, 1.07mol) was stirred and heated at reflux in acetic anhydride ($505cm^3$) for 16 hours. The reaction mixture was cooled and the solid collected by filtration and washed with toluene ($750cm^3$) to yield the 4,5-dichlorophthalic anhydride (177.66g, 77%) as a pale yellow solid, m.p. 183-186°C; ν max (KBr) 1831 (C=0), 1784 (C=0) $cm^{-1}$.

ii) Preparation of 4,5-Dichlorophthalimide

4,5-Dichlorophthalic anhydride (190.12g, 0.88mol) was stirred and heated at approximately 210°C with urea (52.8g, 0.88mol) for 16 hours. The reaction mixture was cooled and the solid was ground and

then stirred in water ($200cm^3$) for 1 hour. The resulting solid was collected by filtration and washed with water ($1000cm^3$) and then dried in air to yield the 4,5-dichlorophthalimide (176.7g, 93%) as a cream solid, m.p. 200°C, ν max (KBr) 3256 (NH), 1781 (C=0), 1730 (C=0) $cm^{-1}$.

iii) 4,5-Dichlorphthalamide

4,5-Dichlorophthalimide (176.2g, 0.818mol) and concentrated ammonia solution ($1825cm^3$) were stirred together at room temperature for 16 hours. The resulting solid was collected by filtration, washed with water (4 litres) and dried at 50°C under vacuum to yield the 4,5-dichlorophthalamide (182.6g, 96%) as an off-white solid, ν max (KBr) 3430, 3304, 3146 ($NH_2$), 1688 (C=0), 1667 (C=0) $cm^{-1}$.

iv) 4,5-Dichlorophthalonitrile

A solution of the 4,5-dichlorophthalamide (182.6g, 0.78mol) in pyridine (1.3 litres) was cooled to 0°C and phosphorus oxychloride ($187cm^3$) was added dropwise over 1 hour maintaining the temperature below 0°C. The mixture was allowed to warm to room temperature and left to stand for 48 hours during which time a brown purple colour developed. The mixture was poured into ice/water (5 litres) and the solid was collected by filtration and washed with ice/water (1.5 litres) and cold methanol (500ml) and then dried in vacuo at 80°C to leave a brown solid. The solid was recrystallised from acetone and water to yield the desired 4,5-dichlorophthalonitrile (120.5g, 86%) as a beige solid, m.p. 178-181°C; ν max (KBr) 2238 (CN) $cm^{-1}$; Found: %C 49.1, %H 1.2, %N 14.3. $C_8H_2N_2Cl_2$ requires %C 48.7, %H 1.0, %N 14.2.

v) Preparation of 4,5-di(4-t-octylphenoxy)phthalonitrile

4,5-Dichlorophthalonitrile (9.85g, 0.05mol), t-octylphenol (22.7g, 0.11mol) and potassium carbonate (15.2g, 0.11mol) in N,N-dimethylformamide ($75cm^3$) were stirred and heated at 120°C for 2 hours and then allowed to cool to room temperature. Water (100ml) was added and the resulting solid was collected by filtration through filter aid (clarcel-flo) and washed with water. The solid was dissolved in dichloromethane ($250cm^3$) and washed from the filter aid, and then washed with water ($250cm^3$), 5% potassium hydroxide in water ($200cm^3$) and then water ($3x200cm^3$), dried ($MgSO_4$), carbon screened and then evaporated to dryness in vacuo to leave a solid. The solid was recrystallised from acetone and water to yield 4,5-di(4-t-octylphenoxy) phthalonitrile (15.59g) as a pale solid, m.p. 168-171°C.

vi) Preparation of octa-4,5-(4-t-octylphenoxy)ironphthalocyanine

The 4,5-di(4-t-octylphenoxy)phthalonitrile (8.52g, 0.016mol), iron (III) chloride hexahydrate (1.42g, 0.0053mol), urea (0.31g, 0.0052mol) and ammonium molybdate (0.0062g, $3.16 \times 10^{-5}$mol) were stirred at 190-210°C for 35 minutes. 1-Chloronaphthalene ($5cm^3$) was then added to mobilise the mixture and heating and stirring was continued for a further 25 minutes after which time the mixture was then allowed to cool to approximately 50°C and methanol ($50cm^3$) was added. The resulting suspension was stirred at room temperature for ½ hour and the solid was collected by filtration and washed with methanol. The solid was then dissolved in toluene and passed through silica gel eluting with a solution of 20% ethyl acetate in toluene. The combined eluent was concentrated and methanol was added to precipitate a solid which was collected by filtration and washed with methanol to yield octa-4,5-(4-t-octylphenoxy)ironphthalocyanine (7.36g) as a dull green solid, m.p. 280-320°C; λmax (toluene) 657nm (ε max 71,000).

vii) Preparation of octa-4.5-di(4-t-octylphenoxy)iron(II) phthalocyanine, bispyridyl complex

A suspension of octa-4,5-(4-t-octylphenoxy)iron (2g) in pyridine ($80cm^3$) was stirred and heated at reflux for 1¼ hours and the allowed to cool to room temperature. The resulting precipitate was collected by filtration and washed with methanol ($25cm^3$) and then dried in air to yield octa-4,5-di(4-t-octylphenoxy)iron(II)phthalocyanine. bispyridyl complex (1.55g) as a bright green solid, m.p. >300°C; λmax (toluene) 658nm (ε max 152000).

The following compounds were prepared using the procedures of Example 1:

| Compound | m.p. | λ max/nm |
|---|---|---|
| Octa-4,5-(4'-fluorophenoxy)-iron(II) phthalocyanine.bispyridyl complex | >300°C | 660 (pyridine) |
| Octa-4,5-(4'-nitrophenoxy)-iron(II) phthalocyanine.bispyridyl complex | >300°C | 659 (pyridine) |
| Octa-4,5-(4'-$^t$octylphenoxy)-iron(II) phthalocyanine.bis-t-butylpyridyl complex | 250-280°C | 659 (toluene) |
| Octa-4,5-(4'$^t$octylphenoxy)-iron(II)phthalocyanine. bis-benzylpyridyl complex | 280-290°C | 659 (toluene) |
| Octa-4,5-(4'-$^t$octylphenoxy)-iron(II) phthalocyanone.bis-4-dimethylaminopyridyl complex | >300°C | 662 (toluene) |
| Octa-4,5-(d,5-di$^t$butylphenoxy)-iron(II) phthalocyanine.bis-pyridyl complex | 270-300°C | 660 (toluene) |
| Octa-4,5-(3,5-di$^t$butylphenoxy)-iron(II) phthalocyanine.bis-4-$^t$butylpyridyl complex | 300-320°C | 661 (toluene) |
| Octa-4,5-(3,5-di$^t$butylphenoxy)-iron(II) phthalocyanine.bis-4-benzylpyridyl complex | 295-305°C | 661 (toluene) |
| Octa-4,5-(3,5-(3,5-di$^t$butylphenoxy)-iron(II) phthalocyanine.bis-4-dimethylaminopyridyl complex | - | 664 (toluene) |
| Octa-4,5-(3,5-di$^t$butylphenoxy)-iron(II) phthalocyanine.bis-4-cyanopyridyl complex | 250-260°C | 660 (toluene) |
| Octa-4,5-(3,5-di$^t$butylphenoxy)-iron(II) phthalocyanine.bis-4,4'-bipyridyl complex | 260-270°C | 660 (toluene) |
| Octa-4,5-(3,5-di$^t$butylphenoxy)-iron(II) phthalocyanine.bis-isoquinoline complex | 270-275°C | 660 (toluene) |
| Tetra-(4-chloro-5-(2,6-di$^t$butylphenoxy)-iron(II) phthalocyanine.bis-pyridyl complex | >300°C | 670 (toluene) |
| Octa-4,5-(2-naphthoxy)-iron(II)phthalocyanine. bis-pyridyl complex | >300°C | 662 (pyridine) |
| Octa-4,5-(2-naphthoxy)-iron(II)phthalocyanine. bis-4-dimethylaminopyridyl complex | >300°C | - |
| Tetra-(4-(2,4-di$^t$butylphenoxy)-5-chloro)iron(II) phthalocyanine.bis-pyridyl complex | 220-230°C | 662 (toluene) |
| Tetra-(4-(2,4-di$^t$butylphenoxy)-5-chloro)iron(II) phthalocyanine.bis-dimethylaminopyridyl complex | 255-270°C | 666 (toluene) |
| Tetra-(4-(2,4-di$^t$butylphenoxy)-5-chloro)iron(II) phthalocyanine.bis-4-benzylpyridyl complex | 240-280°C | 662 (toluene) |

| | | |
|---|---|---|
| Tetra-(4-(2,6-di$^{t}$butylphenoxy)-5-phenoxy)iron(II) phthalocyanine.bis-pyridyl complex | - | 668 (toluene) |
| Tetra-(4-(2,6-di$^{t}$butylphenoxy)-5-phenoxy)iron(II) phthalocyanine.bis-dimethylaminopyridyl complex | - | 673 (toluene) |
| Tetra-(4-(2,6-di$^{t}$butylphenoxy)-5-phenoxy)iron(II) phthalocyanine.bis-4-cyanopyridyl complex | - | 669 (toluene) |

## Claims

1. A phthalocyanine of Formula (1):

$$(R)_aM_kPc(O\text{-}R^1)_b(O\text{-}R^2)_cX_d \qquad \text{Formula (1)}$$

wherein:

$M_kPc$ is a phthalocyanine nucleus of the Formula (2):

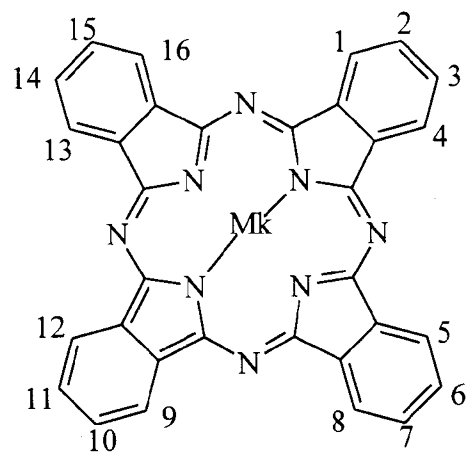

Formula(2)

in which

| | |
|---|---|
| M | is a metal atom; |
| k | is inverse of ½ valency of M; |
| R | is a heterocyclic compound or a tertiary amine; |
| each $R^1$ | independently is an aryl or heterocyclic radical; |
| each $R^2$ | independently is an alkyl or cycloalkyl radical; |
| each X | independently is -H or halogen; |
| a | is 1 or 2; |
| b | is an integer from 1 to 8; |
| c | is 0 or is an integer from 1 to 4; |
| d | is an integer from 4 to 15; and |

$b+c+d = 16$.

2. A phthalocyanine according to Claim 1 in which R is optionally substituted pyridine, quinoline, isoquinoline, bipyridyl or pyrazine.

3. A phthalocyanine according to Claim 1 in which $R^1$ is preferably optionally substituted phenyl, 2-naphthyl or pyridyl.

**4.** A phthalocyanine according to Claim 1 in which
R is pyridine or pyridine substituted in the 3- and/or 4- and/or 5-positions by $C_{1-4}$-alkyl, $C_{1-4}$-alkylphenyl, -CN, or -N($C_{1-4}$-alkyl)$_2$, or quinoline or isoquinoline;
M is $Fe^{II}$ or $Co^{II}$;
each $R^1$ independently is phenyl or naphth-2-yl optionally substituted by one or more straight or branched chain $C_{4-8}$-alkyl group;
εach X independently is -H or halogen;
a is 2;
b is 8;
c is 0; and
d is 8.

**5.** A phthalocyanine according to Claim 1 in which

| | |
|---|---|
| R | is pyridine or pyridine substituted in the 3- and/or 4-and/or 5-positions by $C_{1-4}$-alkyl, $C_{1-4}$-alkylphenyl, -CN or -N($C_{1-4}$-alkyl)$_2$; or isoquinoline; |
| M | is $Fe^{II}$, or $Co^{II}$; |
| each $R^1$ | independently is phenyl or naphth-2-yl optionally substituted by one or more straight or branched chain $C_{4-8}$-alkyl group; |
| each $R^2$ | independently is $C_{1-8}$-alkyl or $C_{4-8}$-cycloakyl; |

εach X independently is -H or halogen;
a is 2;
b is 4;
c is 4;
d is 8.

**6.** A phthalocyanine according to Claim 1 in which

| | |
|---|---|
| R | is pyridine or pyridine substituted in the 3- and/or 4-and/or 5- positions by $C_{1-4}$-alkyl, $C_{1-4}$-alkylphenyl, -CN or -N($C_{1-4}$-alkyl)$_2$, or isoquinoline; |
| M | is $Fe^{II}$ or $Co^{II}$ |
| each $R^1$ | independently is phenyl or naphth-2-yl optionally substituted by one or more straight or branched chain $C_{1-4}$-alkyl group; |
| each X | independently is -H or halogen; |
| a | is 2; |
| b | is 4; |
| c | is 0; |
| d | is 12. |

**7.** A phthalocyanine according to any one of Claims 1 to 6 in which M is $Fe^{II}$.

European Patent Office

# EUROPEAN SEARCH REPORT

Application Number
EP 94 30 5325

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | DATABASE WPI<br>Week 8650,<br>Derwent Publications Ltd., London, GB;<br>AN 86-329625<br>& JP-A-61 246 393 (CANON) 1 November 1986<br>* abstract * | 1-7 | C09B47/08<br>C07D487/22<br>//(C07D487/22, 259:00,209:00, 209:00,209:00, 209:00) |
| Y | EP-A-0 484 027 (ICI)<br>* abstract *<br>* page 6, line 15 - line 19 * | 1-7 | |
| Y | JOURNAL OF THE CHEMICAL SOCIETY, 1938, LONDON<br>pages 1157 - 1163<br>A.BARRETT ET AL 'phthalocyanines and associated compounds'<br>* page 1159, line 26 - line 44 *<br>* page 1162, line 21 - page 1163, line 24 * | 1-7 | |
| A | GB-A-2 260 996 (ICI)<br>* abstract * | 1-7 | TECHNICAL FIELDS SEARCHED (Int.Cl.6) |
| A | FR-A-2 338 950 (BAYER)<br>* the whole document * | 1-4 | C09B<br>C07D |
| A | US-H-477 (W.R BARGER ET AL)<br>* the whole document * | 1 | |
| A | EP-A-0 484 018 (ICI)<br>* abstract * | 1 | |
| A | DATABASE WPI<br>Week 8421,<br>Derwent Publications Ltd., London, GB;<br>AN 84-131212<br>& JP-A-59 067 093 (TDK) 16 April 1984<br>* abstract * | 1 | |

-/--

The present search report has been drawn up for all claims

| Place of search | Date of completion of the search | Examiner |
|---|---|---|
| THE HAGUE | 18 November 1994 | Dauksch, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 94 30 5325

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | US-A-5 132 465 (J.R SANDERSON ET AL)<br>* column 3, line 39 - line 47 *<br>----- | 1 | |

TECHNICAL FIELDS SEARCHED (Int.Cl.6)

The present search report has been drawn up for all claims

| Place of search | Date of completion of the search | Examiner |
|---|---|---|
| THE HAGUE | 18 November 1994 | Dauksch, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)